(19)

**Europäisches Patentamt**
**European Patent Office**
**Office européen des brevets**

(11) **EP 2 392 895 B1**

(12) **EUROPEAN PATENT SPECIFICATION**

(45) Date of publication and mention
of the grant of the patent:
**06.03.2013  Bulletin 2013/10**

(51) Int Cl.:
***G01B 11/24*** *(2006.01)*     ***G01B 11/06*** *(2006.01)*

(21) Application number: **10164636.2**

(22) Date of filing: **01.06.2010**

(54) **Method for measurement of geometrical parameters of coated threaded joints**

Messverfahren und Messvorrichtung für beschichtete Gewindeparameter

Procédé et dispositif de mesure de paramètres de filetage avec revêtement de surface

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(43) Date of publication of application:
**07.12.2011  Bulletin 2011/49**

(73) Proprietor: **Tenaris Connections Ltd.**
**Kingstown (VC)**

(72) Inventors:
 • **Bonadeo, Nicolas Hernan**
**B2804MHA BUENOS AIRES (AR)**
 • **Berra, Sebastian**
**B2804MHA BUENOS AIRES (AR)**

 • **Etcheverry, Javier Ignacio**
**B2804MHA BUENOS AIRES (AR)**

(74) Representative: **Cinquantini, Bruno et al**
**Notarbartolo & Gervasi S.p.A.**
**Corso di Porta Vittoria, 9**
**20122 Milano (IT)**

(56) References cited:
**US-A- 5 521 707**

 • **"LASERS GAUGE PITCH" MACHINE DESIGN, PENTON MEDIA, CLEVELAND, OH, US, vol. 67, no. 19, 26 October 1995 (1995-10-26), page 40, XP000545874 ISSN: 0024-9114**

EP 2 392 895 B1

**Description**

**Field of the invention**

The present invention relates to a non-destructive method for the measurement of geometrical parameters of coated threaded connections and for assessing the quality of a coating deposition process. This method is especially suited for threaded pipes used in the hydrocarbon industry and for similar threaded objects.

**Background of the invention**

Pipes with dry coatings applied in oil and gas pipe connections are already employed in the art to avoid the use of dopes and the drawback caused by it. Documents EP1554518, EP1954953 and EP2102542 disclose threaded joints where all or part of the threading is covered with dry coating.

Generally dry coatings are applied after performing a threading operation of pipe ends and must assure both high galling resistance during make up operations in the oil field and high corrosion resistance, this latter feature being necessary during transport and storage of the pipes so that they are not damaged. Independently of the fact that a threading is coated at a later stage of the production process or the metal surface of threading is left uncovered, during a thread manufacturing process such as that of a screw, bolt or of a threaded pipe, it is necessary to verify that the geometrical dimensions of the piece comply with the tolerances set for the product. In addition, knowledge of the nature of the deviation from these tolerances can be used for feedback to the manufacturing process, so avoiding rejects later on in the process.

A main problem in performing the measurement operations for quality control is the precision and repeatability of measurements. In the past there have been attempts to improve accuracy and repeatability of the measurement operations and to make measurement systems capable of measuring the thread shape of complex mechanical objects like the threading of pipes used in the oil industry. In this particular technical field there is the need to measure several parameters like taper of pin and box, thread pitch, thread height, pin or box diameter, pipe ovality, run in and run out.

For instance document US5521707 discloses a non-contact laser-based sensor guided by a precision mechanical system which scans a thread profile and produces a set of computer images of the threading. The computer images are then analyzed to acquire quantitative information about thread characteristics such as pitch, lead, root radius, flank angle, surface roughness, helix variation, and pitch diameter. That document however has the disadvantage that it does not address explicitly the important problem of piece misalignment and therefore it requires an absolute precision of the operations when aligning the piece to be measured with the mechanical system coordinates. This alignment can only be achieved when the piece is at the threading machine. Measuring at the threading machine has several disadvantages; it adds costly time to the threading process by preventing the inspection and manufacturing process from running in parallel, it requires placing delicate optics and precise mechanical components in a hostile environment with cutting oil and strong vibrations present, and to some extent uses the same mechanical movement that has to be verified. Once the piece has been removed from the lathe, this alignment is very difficult to achieve manually and consequently the system disclosed by that document only allows measurement of relative or local magnitudes, i.e. thread height by comparing contiguous crests and roots, whereas errors introduced by a piece misalignment are not "noticed" by that solution and in these cases they produce an insufficiently precise measurement. It also does not address the measurement of important thread parameters such as taper, run-in, run-out, black crest, length of complete thread or specific process parameters such as taper profile, pitch linearity, Fourier mode decomposition of ovality, lathe plate misalignment, hook end angle severity to name some.

In case the pipe threading must also be coated, eg. with a dry lubricant, additional problems pop up. As in the case of non coated pipes, it is very important to ensure and verify the geometrical dimensions of the finished piece after the coating process, in order to comply with the tolerances set for the final product with the threading. In addition, the information on the nature of the deviation from these tolerances can be used as a feedback on the manufacturing process, so as to avoid rejects later on in the production process.

The problem of ensuring precision and repeatability of measurements is paramount also in this case, as in the case of non-coated threadings, whereas another important problem in performing the measurement operation specifically when the joints are coated is the fact that state of the art measurement methods cannot ensure that the coating material is not damaged during measurement procedure, eg. due to handling of the pipes and to the use of contact type measuring devices.

In the known state of the art there are some measuring systems which are proposed for measuring coatings in general and few of them refer to measurement of coating applied on tubular products. However, none of them is especially adapted to measurement of dry coatings applied on threaded parts of tubular joints.

A measurement technique using ultrasounds is known, but it has the drawback that it cannot be applied to coatings with small thicknesses as those applied in threaded joints for the hydrocarbon industry, since the wavelength of the ultrasounds is much larger than the thicknesses to be measured.

Another measurement technique uses eddy currents, but it has the disadvantage that the measurement device must be placed either in contact or very close to the work piece. It is difficult to use this technique on threaded parts of joints because of the complex geometry and because of the generation of boundary effects. The deformation of field lines because of the geometry and the fact that the sensor must be very near to the thread surface are two important constraints.

Still another known measurement technique is based on X-ray fluorescence or back scattering, whereby the coating highlights when it is irradiated and the fluorescence is reabsorbed by the coating. Thus the amount of fluorescence measured is proportional to the thickness. The results are influenced by several factors. It is not a technique generally applicable and, in complex cases, the results depend on the angle of incidence of X-rays. Another important drawback is the use of X rays which are harmful to operators.

A similar measurement technique is based on infrared (IR) absorption, where excitation of the coating is made by means of visible light. Its application depends on whether the coating is made of a material which is excitable by light and on the grade of IR absorption.

Therefore the need is felt to provide a measurement device and method of general use on threaded joints by means of which measurements can be made in a repeatable, satisfactory and sufficiently precise manner.

**Summary of the invention**

It is therefore an object of the present invention to provide a measurement method which overcomes the aforementioned drawbacks and limitations.

A main object of the present invention is to provide a method for accurate, automatically performed, non-contact inspection of threaded objects, in particular of oil pipe threads, especially of pins and boxes.

The above mentioned objects and others which will become apparent in view of the following description, are achieved according to the present invention, by means of a measurement method according to claim 1 for thread parameters of a threaded object coated by at least one layer.

In the following description we refer to trajectory as the path a sensor follows through space, describing a sequence of values of the surface location with respect to the sensor.

The method according to the invention achieves several advantages:

- its use on coated threaded joints provides measurements which are precise and performed automatically. This is combined with the advantage of being a non destructive method as there is no contact between the coated surface and the measuring instrument, whereby the eventuality of damaging the coated surface during the measurement operation is drastically minimized, because of the use of non-contact sensors, like laser sensors or other optical sensors.
- it allows acquisition of quantitative information about thread characteristics, e.g. taper, seal diameter and ovality, run-in, run-out, thread diameter and ovality, pitch along a plurality of generatrices of the tube, and step height,
- it allows absolute measurements to be performed on several threading parameters and tube features regardless of any misalignment between the threaded tube and the measurement apparatus,
- the object to be measured is precisely located in space by means of the series of scanning and fitting steps performed, regardless of its position and alignment with respect to the measurement device.

It will be appreciated that an important feature of this method is the fact that the thread frame of reference is detected independently of the position that the threaded object has in respect of the measurement device and the condition of coated and uncoated surface. This feature allows following the same trajectory when performing the measurement process before and after the coating operation.

According to the invention there is also provided a device according to claim 19.

Therefore, not only the geometrical parameters of coated threaded objects can measured but also the quality of a coating deposition process can be verified and assessed.

Over state-of-the-art methods that can measure coating thickness but are not designed to measure the dimensional tolerances of the thread the method of the invention solves both problems, measuring the coating NDT plus the final dimensional tolerances of the thread.

**Brief description of the figures**

The present invention will be now described according to a preferred embodiment thereof, which is given by way of non-limiting example, by means of the accompanying figures where:

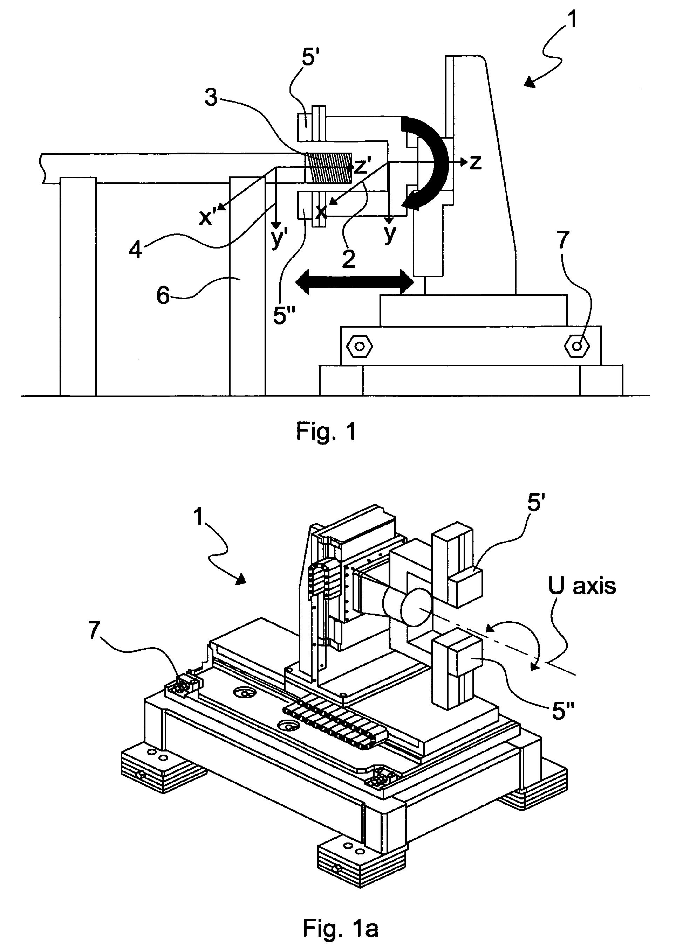

Figure 1 shows a schematic axonometric view of a measurement device for implementing the method according to the invention,
Figure 1 a shows a perspective view of a measurement device to perform the method in accordance with the invention. ,
Figure 2 is a flow chart showing the scheme of the measurement method of the invention,
Figure 3 is a graph showing results of a step in the data acquisition procedure,
Figure 3a shows a detail of a coated threaded object on which the measurement method of the invention is performed,
Figure 4 is a graph showing a further step in the data detection procedure,
Figure 5 shows a scheme of a thread longitudinal section where measurements are made,
Figure 6 is a graph showing results of further steps in the data acquisition procedure,
Figure 7 are graphs showing hypothetical assignations of load flanks,
Figure 8 are graphs showing results of spiral scans,
Figure 9 are graphs showing results of seal scans,
Figure 10 shows a scheme of the threading on which the method of the invention is used,
Figure 11 are graphs showing results of thread root scans,
Figure 12 are graphs showing results of thread crest scans,
Figure 13 shows schemes of the threading on which the method of the invention is used,
Figure 14 are graphs showing results of thread run-in scans,
Figure 15 are graphs showing results provided by the measurement method of the invention,
Figure 16 shows the step height calculation on a thread using linear fitting of crests and roots.
Figure 17 is a graph showing results of another thread scan.

**Detailed description of preferred embodiments of the invention**

With particular reference to the figures 1 and 1a, there is shown a scheme of an automatic thread inspection optical system based on laser displacement sensors, called in short hereafter "measurement" device 1, which has its own Cartesian reference system 2, defined by the orthogonal axes X, Y, Z. A threaded portion of a pin 3 to be measured is shown on the left of the figure. This pin has its own Cartesian reference system 4 defined by the orthogonal axes X', Y', Z'. In the figure 1 the pin is positioned far from the measurement device in a rest position on a bench 6.

In the following description of the invention, for the sake of simplicity, reference is made to a pin of an oil or gas pipe, however it is understood that the invention can have an application also for any other similar threaded objects, like screws or similar objects. All these kinds of objects, at a certain stage of the measurement method, are coated with a dry lubricant layer, using any type of coating technology, but also other types of materials used as coating can equally be employed, depending on the function to be achieved by means of the coating. The coating is not shown nor indicated in the figures appended because of their schematic character and of the small thickness of the coating layers, in the order of the microns, but it is understood that the figures apply also to the cases where a coating is present on the measured object.

When initial installation of the device is performed, a pin 3 is mounted on the measurement device 1 to perform the measurement operations according to the invention, the respective pin and measurement device 1 reference systems are placed as close as technically possible one to each other using the horizontal and vertical angular and lateral displacement movements provided by the device (see fig1). Nevertheless, despite all possible care, the two reference systems will not coincide exactly and small misalignments, both in the angular and in the lateral position of the reference system 2 of the pipe, with respect to the measurement device may still exist. Even more, when another pin 3 is positioned, its angular and lateral position will differ from the previous pin due to placement errors and geometrical differences (such as hook end) between the pieces. Due to these reasons, it is generally the case that under operative conditions the misalignment of pins, or more generally of the threaded parts of pipes, with respect to the measurement device, is of the order of millimetres in the linear displacement and in the order of a degree in the angular displacement. Only by adjusting the position of every pin a smaller misalignment value could be achieved, however, that scenario is impractical in reality. The invention achieves the objective of the device working without any lateral or vertical adjustment of the knobs 7, shown in figures 1 and 1a, after initial installation.

The measurement device 1 comprises two laser displacement sensors 5' and 5" mounted on a yoke piece advantageously machined from a single aluminium piece to minimize mechanical movements. This piece or head is mounted on a rotational stage, able to pivot about a U-axis, and belongs to the head of the measurement device, together with the laser sensors 5' and 5". Each sensor 5', 5" is mounted on an X stage that can be moved in the radial direction i.e. the X-axis, by means of linear rotary motors. Angular and linear movements of these stages parallel to the Y axis, orthogonal to the X axis, allow the stage to be aligned along the same line. Both laser sensors 5' and 5" can also be adjusted by lateral and angular displacements to align the center of the laser emission with respect to the center of rotation of the device.

The stage X in addition can be motorized so that it can be used for an automatic change of product diameter while maintaining the sensors always within their range.

Finally, there is provided a linear stage, (moving along the Z-axis) that allows displacing the head parallel to the device axis Z. The linear stage, movable along the Z axis, is mounted on a base that is hardcoated on the bottom to allow smooth displacement with respect to the base when the device is set up for the first time, as shown in figure 1a. Four knobs, e.g. placed one on each corner, allow for horizontal angular and lateral displacement in a plane. There are provided screws, or equivalent means, to fix the whole structure to avoid movement once the initial alignment has been accomplished. There is provided advantageously a wedged pad on each of the four legs to regulate height and out-of-plane angular alignment.

All these regulation possibilities provide easy initial installation of the device in a plant, allowing for small corrections to levelling tolerances, to conveyer alignment and to imperfections in the levelling of the plant floor.

The manner in which this measurement device is used to perform measurements on threaded objects is described hereafter. After the initial installation is carried out, as described above, a typical measurement operation comprises two main parts: data acquisition and data analysis.

The data acquisition procedure is now described. During this procedure the laser sensor output signals and positions of servos are stored in a synchronized manner ensured by the use of hardware signals to enable windows and counters. Below, a description is given of the signals used.

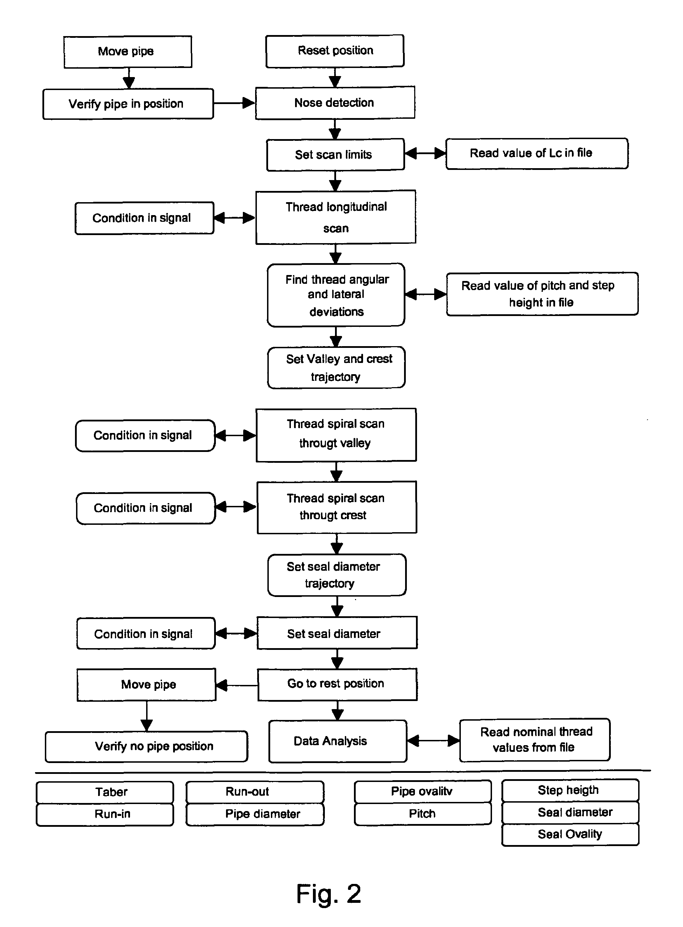

Figure 2 shows, by means of a flowchart, the general working scheme of the data acquisition procedure governing the whole measurement process once the initial calibration and plant installation has been performed. It starts by verifying that the pipe is in position aligned to the measurement device, then the measurement device executes a first longitudinal scanning operation along the threaded portion of the pipe to find the relative position of the pipe nose with respect to the frame of reference of the measurement device. After the relative position of the nose is detected, all distances reported during measurement are referred to that point. If the measurement operation of the threaded pipe provides for only one scanning operation, the data are gathered only in correspondence with points belonging to the path followed by the sensors 5' and 5". This is generally done because the data collected are considered sufficient to the needs of the users.

If the measurement procedure on the threaded object provides for several scanning operations along several trajectories on the coated or uncoated surface of the threaded portion, data are also collected on predefined points along these trajectories. The choice of the measurement points where data are gathered is made in such a manner that a matrix that describes the quadratic form has maximum rank when values corresponding to these points are inserted in it.

In a preferred embodiment of the method according to the invention, a plurality of longitudinal profiles, e.g. six, are scanned at equally spaced angular steps. Data gathered by means of these scans are processed to remove spurious peaks and pass encoder counts to physical units and are then used to calculate a first estimation of the thread angular misalignment with respect to the measurement device frame of reference and reassign the nose position by averaging the six nose positions corresponding to the six scans.

These scanning operations are also used to detect the positions of the crests and roots of the threads and define a spiral trajectory table, by interpolating this information, so as to allow successive scanning operations over the center of the crests or roots of the thread. Data acquired by means of those spiral scanning operations are also conditioned and used to measure more precisely the misalignment in respect of the thread frame of reference. After this operation, and having detected the orientation of the thread, a table for the seal or seals, run in and run out trajectories, can be constructed in the reference frame of the piece (X',Y',Z') and transformed to the device coordinate system and executed.

After the end of the scanning operations, the head of the measurement device returns to its rest position and data analysis begins in order to obtain all the parameters of the thread under test.

The pin 3 is then dismounted from the device 1 and, at a subsequent moment in time, a coating operation of the threaded object is performed in an appropriate place. One or more layers of coating material are laid on at least part of the pin threading and/or surface. The coating can be made also on specific surfaces of the threading, like thread crests, flanks or roots by means of any known technology.

When coating operation is performed, the pin 3 is again mounted on the device 1 or any similar device and the measurement steps described above are replicated on the same predefined trajectories on the surface of the threaded portion, and data are again collected on the same predefined points along these trajectories, which were selected before the coating operation, and the same calculations, as already described, are again performed. Consequently a comparison of the data acquired before coating and after coating is carried out and, in this manner, the thickness of the coating at all measurement points of the pin is calculated.

The procedure described before in broad terms is hereafter described in detail by splitting it into several steps and indicating various embodiments of the measurement method of the invention for calculating a series of specific parameters of the threading. This procedure applies to both stages, before the pin is coated and after the pin has a coating laid on its surface, and it can be appreciated that the measurement method of the invention can be advantageously applied to the cases where coating is made in successive distinct layers, to measure the quality of the intermediate layers and of the final layer resulting from the superposition of various layers.

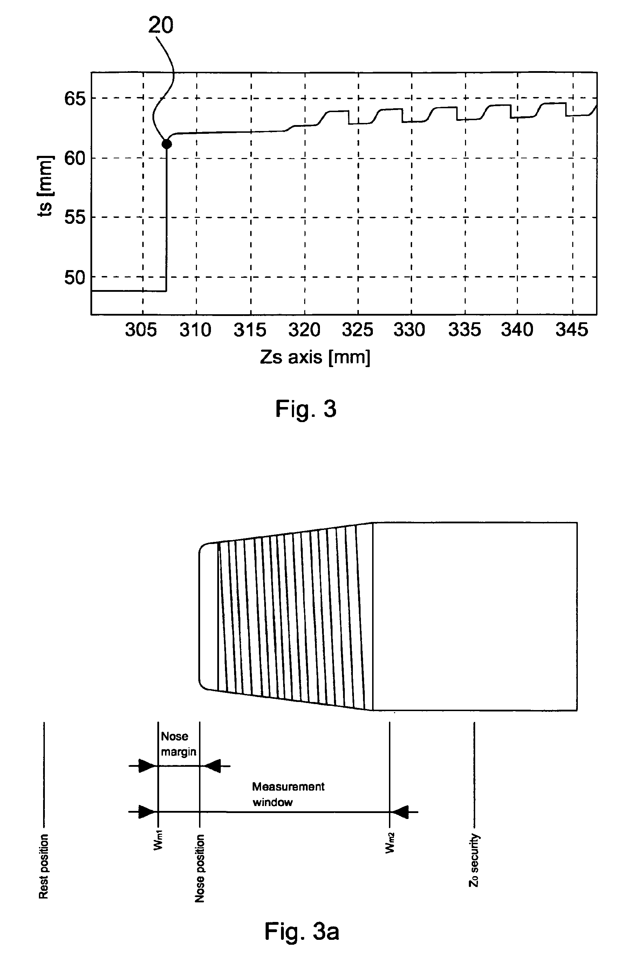

The first step of the data acquisition is nose detection, which consists in executing a linear scan along the Z-axis between two reference distances where the nose is estimated to be located. It will be appreciated that this linear scan can be the only scanning operation predefined in the measurement method, or it can also be the first scanning operation of a plurality of successive scanning operations. The signals provided by the laser sensors 5 are further analysed by making a check of the presence or absence of Out Of Range (OOR) values. An OOR value consists of a non-valid point (i.e. out of the physical range of the sensor), as sent by a sensor when no object is found in the measurement range of the sensors. These signals are processed by defining a sampling window of e.g. fifty data points and verifying that all samples are not OOR. The number of data points depends on several factors like the shape of the pin surface, the type of threading, the type of joint, etc. and can certainly be either above or below fifty. The sampling window is then moved one step further and the values of the signals sent are checked again until all samples in that window are recognised as valid data points after processing. The first sample of that block is defined as the nose position of the pin. The graph of Figure 3 shows the results produced by a scan during nose detection. It shows OOR values previous to the encounter of the laser sensors with the pipe nose, the dot 20 indicating the position, with respect to the Z axis, where the nose has been detected. After this point has been detected, scanning is stopped, and the following step of the data acquisition procedure is started.

An accurate determination of the nose position is not necessary for measuring most of the thread parameters of interest that are based on relative distance measurements, except in the case when it is necessary to measure pipe and seal diameters. These parameters are measured at a precise distance relative to the nose position because the thread taper changes its values if measured elsewhere. After the position of the nose has been determined, in those cases where this detection is necessary, several longitudinal scanning operations, e.g. three (but a greater or smaller number are possible), are performed along the Z-axis direction by acquiring outputs at the same time from both laser sensors 5', 5". A definition of the limits of the scanning range window is represented generically in Figure 3a . From its resting position, the head of the measurement device is driven back to the first position of the measurement window indicated by Wm1, corresponding to the nose position, which is at the end of the nose margin.

When the sensors are placed in position Wm1, a movement towards security point Zo is indicated and the window signal that resets encoder counts is enabled. The number of encoder counts to be acquired by the laser sensors is preset to fit into the measurement window longitude. When this preset number is reached, the head of the measurement device is commanded to stop at the point indicated by Wm2. In this manner, the measurements from the laser sensors 5', 5" together with encoder counts are acquired in the measurement window segment. Longitudinal scans are subsequently implemented in the inverse direction. Similar limits for encoder counts to be acquired by the laser sensors are set also for these movements in the opposite direction. This movement in the reverse direction towards the rest position starts from point Wm2 and ends in point Wm1, where it stops after having reached the predetermined encoder counts to fit the measurement window.

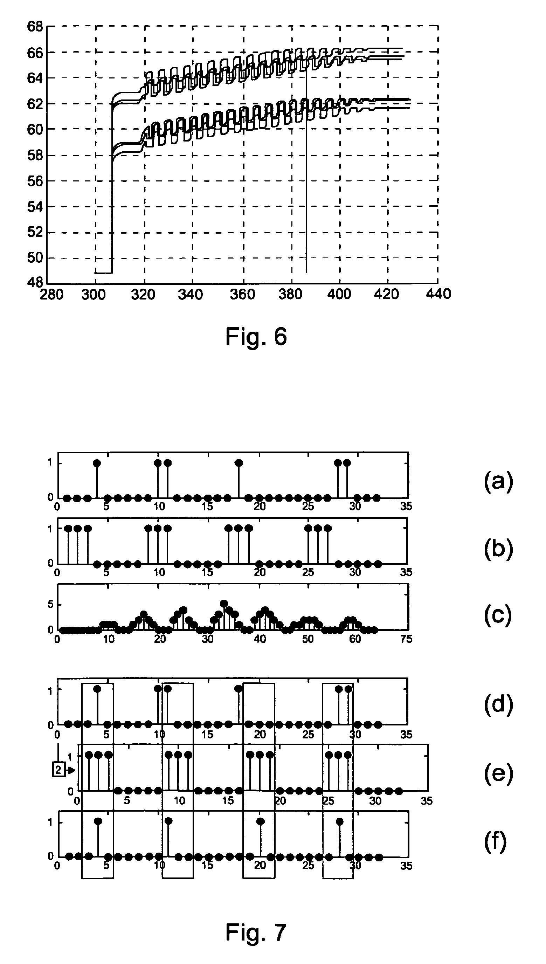

Figure 6 shows a typical longitudinal scan where signals generated by both laser sensors 5', 5", placed at an angular distance of 180° apart, are acquired. In this example the three scans result in six profiles, i.e. two groups of three profiles, each corresponding to one of the laser sensors 5' and 5", of the thread under test, and are useful for giving a first estimation of the thread misalignment. If necessary, the device of the invention can be operated with only one of the two laser sensors 5', 5" generating signals.

The quantity of scans, given here by way of example, can also be more or less than three, depending on the object to be measured and depending on the parameters that are sought. In these particular cases, pitch and step height along six generatrices are the parameters sought.

Another variant of the measurement method provides for a data acquisition operation consisting of detecting selected points on thread crests and roots made by performing a number of longitudinal scans parallel to the Z axis. Data collected from these scans also enable points to be determined on thread roots and crests that are used for the definition of trajectories along which two subsequent spiral scanning operations are performed, one along the thread crest and the other one along the thread root. By being generated previously in this manner, the trajectory avoids falling off the crest or climbing out of the root during the scanning operation when the object is misaligned with respect to the X', Y', Z' co-ordinate system.

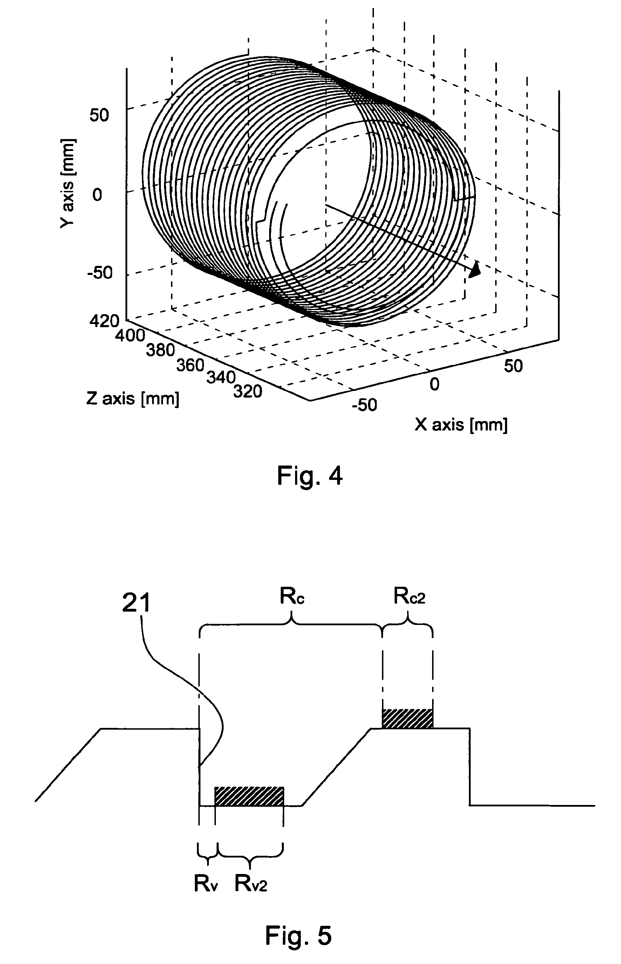

The first step of this measurement operation starts by detecting thread load flanks, consisting of detecting load flanks for each longitudinal profile. This is performed by differentiating the whole data vector and evaluating values that override a preset threshold. A vector containing all zero values is generated, excluding those detected points which are candidates for indicating the presence of a load flank. Another vector is generated which represents a theoretical comb with teeth of a specific detection width and nominal pitch separation between the teeth. These two vectors are cross correlated in order to find the relative position between the comb and the load flank 21 candidate vector that maximizes the cross correlation (see fig. 5). The cross correlation consists of performing a scalar multiplication of the two vectors and finding the sum of the resulting vector while changing their relative vector index.

Subsequently load flanks 21 are assigned in correspondence with the points found as candidates for each comb tooth according to the following criteria:

- If one candidate flank is present (which is defined as type 0): this flank is a real load flank.

- If no candidate flank is present (type 1): a flank is created just in the middle of the thread comb interval for the purpose of producing the spiral trajectory.

- If more than one candidate flank is present (type 2): there are spurious flanks in the thread comb, so the nearest to the middle point of the thread comb interval is determined as the real load flank. The remaining ones are dismissed.

- If an out of range is found in the interval (type 3): the flank is discarded and

a virtual flank is created for the purpose of producing the spiral trajectory.

Figure 7 shows hypothetical load flank candidates and comb vectors in the three mentioned cases possible for assigning loading flanks.

Another variant of the measurement method provides for assigning points along crests and roots, wherein the crests and roots segments are defined following the thread mechanical drawings with the parameters Rc, Rc2, Rv and Rv2 as shown in Figure 5 .

Each segment is conditioned by filtering OOR and possible peaks taking into consideration that the segment should be a line. Then, the point corresponding to either root or crest is calculated as the mid segment point.

Figure 7 shows a longitudinal scan where those points detected as being root and crest points are indicated. Graph (a) shows the candidates for load flanks, graph (b) shows the theoretical comb, graph (c) shows the cross-correlation, graph (d) shows candidates for load flanks, graph (e) shows the displaced theoretical comb, graph (f) shows assigned load flanks. As can be seen in this Figure, points are generated by extrapolating the determined points in the threaded portion for location prior to the nose position, and after the end of the thread where the non-machined part of the pipe starts. This is done to smoothly enter and emerge from the threaded portion during execution of the spiral scan, to obtain the phase of the thread relative to the measuring device frame of reference, to estimate the position of the black crest, to calculate the lathe eccentricity axis, and to measure the pipe hook end.

All the root points determined in each longitudinal scanning operation are also used for fitting a quadric surface representing the cone of the thread being analysed so as to gather a first estimation of the thread angular misalignment with respect to the measurement device reference frame. Crest points are preferably not used in this calculation for two main reasons:

a) the determination of these points is more inaccurate than that of root points, (i.e. the portion of the pin where determination of the roots is performed is longer) and
b) the number of root points over the thread surface cone is higher than that of the crest points due to the presence of "black threads" generated in the manufacturing process.

The generic matricial form of the quadric surface is described in formula [1].

$$\bar{x}^t . A \bar{x} + \bar{b} . \bar{x} = 1 \tag{1}$$

where $\bar{x} = [x, y, z]'$
is a point of the quadric in the 3D space, A is a symmetric matrix related to the quadric (it is formed by nine parameters, three for translation, three for orientation and three for the quadric form as expressed in a canonic frame) and $\bar{b}$ is the quadric displacement vector.

The selected data is fitted to the expression shown in [1] by using a least squares approximation, from which the parameters of the quadric (e.g. the parameters that conform to A and b) are obtained.

These data are useful for the conformation of a linear transformation and its inverse transformation between the measurement device and thread reference frames.

The subsequent operation consists of spiral scannings, both along root and crest of threads. The information acquired before relating to the root and crest mid point positions, for each longitudinal scan, is interpolated and used to build two spiral scanning tables. All of the root mid points are transformed to the thread coordinate frame. Afterwards, a linear fit is performed over the data resulting from a t vs. Z arrangement. Subsequently, a new set of points is generated segmenting the fitted line with a regular step. These points are transformed back to the measurement device reference frame and passed to a controller for the calculation of the servo references. The same procedure is applied on the crest mid points for generating the crest scanning table.

Preferably, the root scan is executed starting from the nose while the crest scanning is executed in the opposite direction, considering the Z-axis. Figure 5 shows a typical root and crest scanning expressed in respect of the measurement device reference frame. Data obtained from the root spiral scan are used to calculate a better estimation of the measurement device to thread misalignment that is also used to recalculate the transformations between reference frames that are applied in the data analysis.

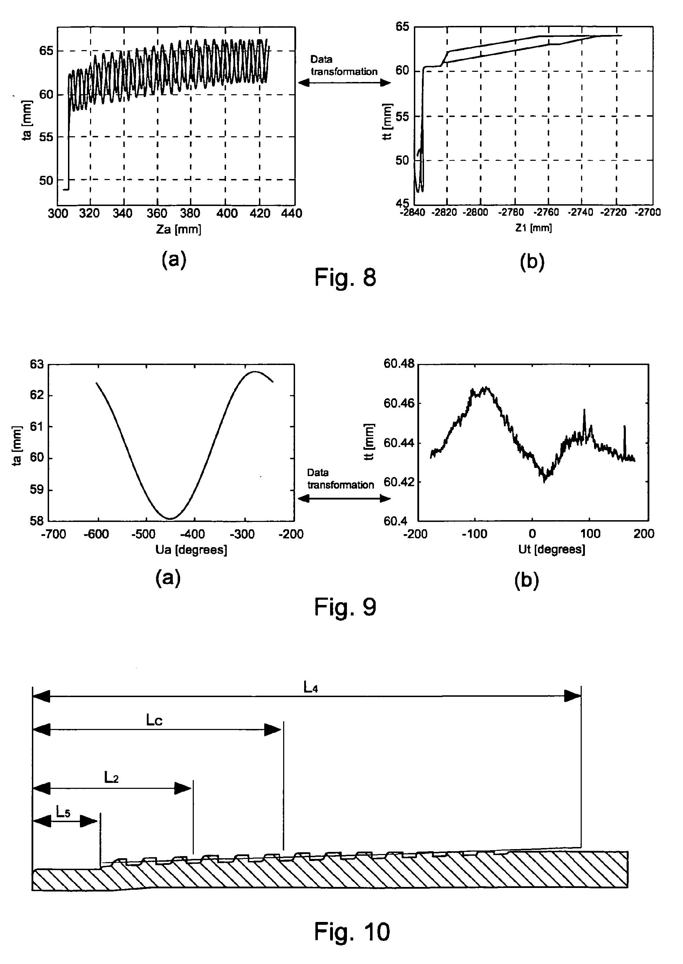

Figure 8 shows the t vs. Z graphs for the measurement device reference frame, graph (a), as data are acquired and for the thread reference frame, graph (b) transformed using the misalignment estimation calculated from the spiral scans. The graph (a) of figure 8 shows on the left what the effects of misalignment are on the acquisition procedure.

In a specific embodiment of the measurement method a data acquisition procedure provides for scanning the seal of the thread. In this case, an ideal circular trajectory is generated with reference to the thread reference frame at a predetermined distance from the nose and then transformed to the measurement device reference frame for its execution. Figure 9 shows two graphs containing the results of a typical seal scan, where graph (a) shows the scan expressed in the measurement device reference frame and graph (b) shows the scan in the thread reference frame.

This scanning trajectory must be executed starting at a predefined distance from the pin nose and in alignment with the threaded object because the nose profile may be complex and the radius measured may be highly dependent on the exact position measured. A good estimation of the nose position is calculated when a plurality of scans is made, e.g. six. In this example, the nose position detected on each of the six longitudinal scans is retrieved. These data are then transformed to the thread reference frame and averaged to get a single, more accurate, nose reference.

In another embodiment of the measuring method the phase of the thread with respect to the device reference frame is retrieved from the spiral root scan and a longitudinal trajectory is set in the thread reference frame such that it passes through the measuring points determined in the inspection report for the measurement of the run-in. Similarly, a trajectory is set for the points defined for the measurement of the run-out.

After data acquisition operations are completed, data analysis is performed on the acquired data, but transformed to the thread reference frame, as a final part of the measurement method according to the invention. Unless indicated differently, in the following all data are expressed on the threaded object reference frame.

Most of the parameters are indicated in relation to different length references taken on the thread and referenced to the nose position. Figure 10 shows the three main references used in data analysis that will be referred to below.

L5 is the length to the beginning of the thread;
L2 is the reference length for the thread diameter and ovality calculation;
Lc is the minimum length where the thread parameters must fulfil the tolerances;
L4 is the length to the end of roots and crests, which is lower than the pull-out length where the run-out is measured.

Determination of the taper is one operation of data analysis. The equation that describes the outer surface of the cone thread is the following.

$$t_{thread} = \left( R_0 - A \bullet Z_{thread} \right) \qquad [2]$$

Where $t_{thread}$ and $Z_{thread}$ are the radial and azimuth coordinates in the thread frame of reference,
Ro is the primitive radius and A is the taper of the pin threading.

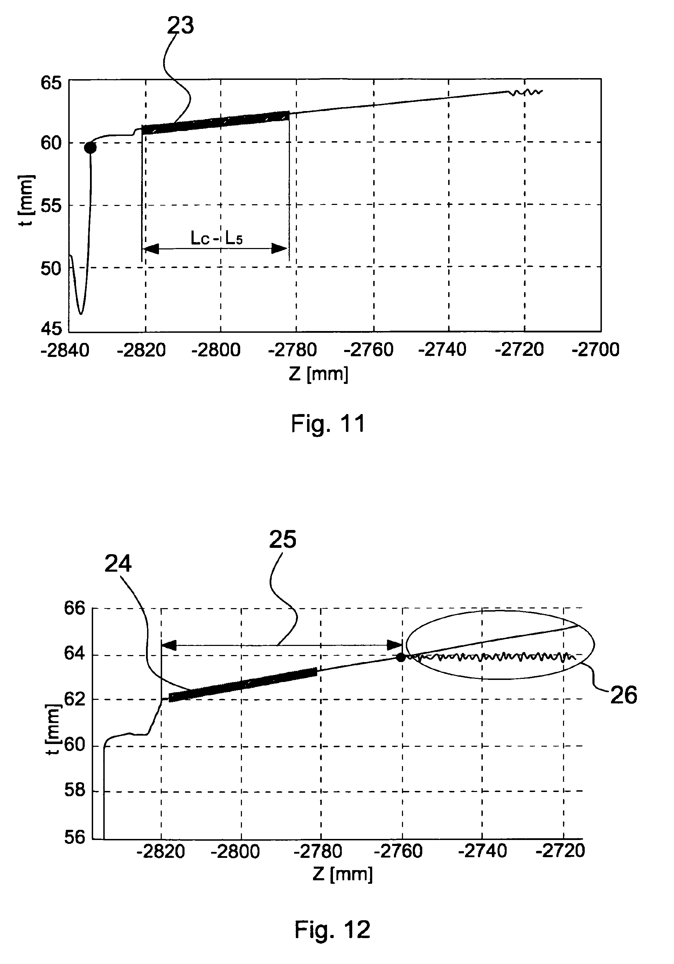

To calculate the taper, the t vs. Z relationship from the root scan of the thread is used. Data acquired previously between L5 and Lc, corresponding to segment 23 of the curve, is analyzed as shown in Figure 11. A linear fit is performed over these data 23 to calculate the slope, comprising the values of taper and of A. The deviations of the data with respect to that linear fit are calculated. Those deviations contain information regarding the machining process, e.g. the non-compensated forces due to the change in pipe stiffness along the thread object and the over tightening of the pipe on the lathe. This information can be retrieved using a Fourier modal analysis as a function of the thread position. For example a large three-mode means overtightening of the pipe on the lathe, while a parabolic behaviour of the fundamental mode means that the tool was taken before the end of the Lc.

Full thread length, corresponding to the segment 25 of the curve of figure 12, is analysed considering the data gathered from the crest scanning. First a linear fit is performed using the acquired, and filtered, data between L5 and Lc, corresponding to segment 24. Deviations of the data with respect to the linear fit are calculated. Subsequently those values over L5 that are more than 0,1 mm greater than this linear fitting are identified, corresponding to segment 26 of the curve in Figure 12. With these values, a new linear fit (using the Z positions and the errors) is performed and the zero abscissa is calculated. This value is defined as the upper limit for the full thread length as shown in Figure 12. Further analysis can be done using the non-machined part of the pipe shown in figure 12. These values correspond to the pipe with a "virgin" surface and can provide information on the position of the thread with respect to the pipe at the moment of machining the part. For example, if the lathe has its plate misaligned, the thread will be off-axis with respect to the pipe, or if the pipe has a hook end the pipe and thread axis will not be parallel. Those variables can be easily calculated by finding the transformation between the pipe and thread coordinate systems.

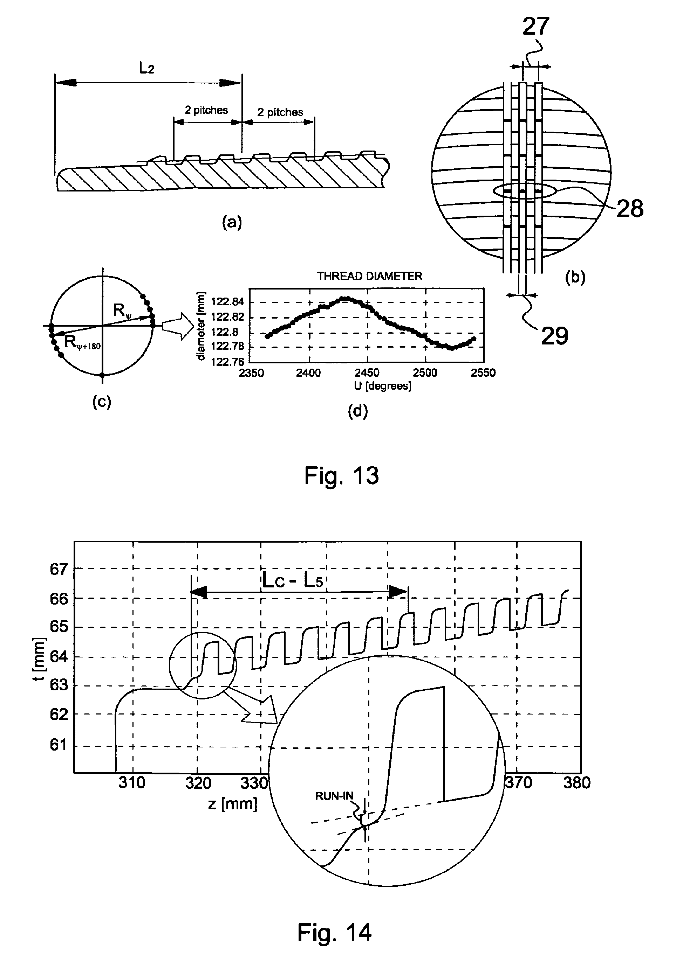

Another data analysis operation relates to calculation of thread diameter and ovality. Thread diameter and ovality are evaluated in two ways using the root spiral scan. The data being analyzed are those comprised in the zone corresponding to L2 $\pm$ 2 thread pitches. Root points defined in this zone, cf. Figure 13 (a) showing the thread lateral view in section , are linearly fitted, after which this fitted linear function is evaluated to obtain the radius.

Alternatively, root points for each radius determination are performed on generatrices equally spaced, advantageously at angular distances 27 of three degrees. On each generatrix, data for each root are averaged considering a generatrix width 29 of typically, but not necessarily, 5 degrees, cf. Figure 13 (b) showing a thread top view. This implementation is similar to the mill procedure used with the MRP gauge.

Radii of defined points, cf. circle 28 of fig. 13(b), are calculated for generatrices ranging from 0 to 180 degrees, with steps of 3°, together with the opposite generatrices for diameter calculation as shown in Figure 13(c), illustrating a thread frontal view. Successively thread diameter and ovality are calculated by means of the following relationships:

$$Diameter = (D_{max} + D_{min})/2 \qquad\qquad [3]$$

$$Ovality = (D_{max} - D_{min})/2 \qquad\qquad [4]$$

where $D_{max}$ and $D_{min}$ are the maximum and minimum diameters calculated through this process respectively. The results are shown in the graph 13(d).

Another operation in the data analysis procedure is run-in evaluation and this is derived from a longitudinal scan done in respect of the thread reference frame.

Figure 14 shows one of these scans which details the region in which the run-in is calculated.

First, a linear fit is performed with unfiltered root data points between L5 + pitch and Lc. This fitted line is compared with the root data included in the segment defined by Rv and Rv2 as shown in Figure 5 and referred to L5. A linear fit is performed over the error array resulting from that comparison. Then, the difference between this fitting, evaluated at the root mid segment value, and the previous fitting is defined as the run-in value.

Subsequently a run-out analysis is carried out in a similar manner to the operation performed for the run-in.

Yet another operation is a pitch determination wherein the load flanks generated from the longitudinal scans are analysed.

The vectors containing the load flank values for each longitudinal scan are truncated so as to keep the flanks between L5+pitch and Lc. Of these flanks just the flanks of type 0 are accepted. The remainder of the types are discarded, being considered unreliable as real load flank identifications.

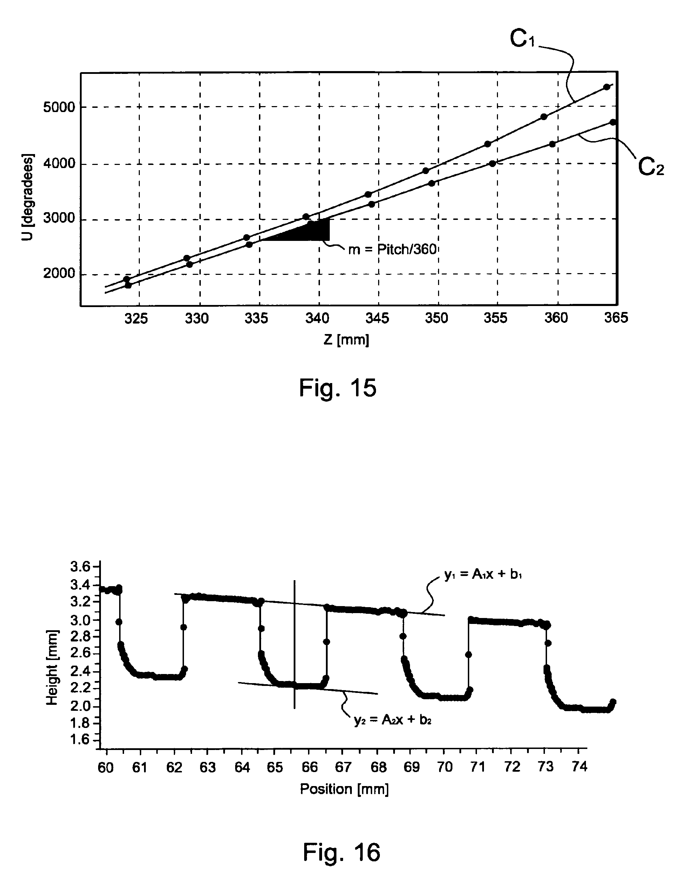

Figure 15 shows a hypothetical longitudinal scan and the positions of the load flanks identified. The curve $C_1$ is how this would be if expressed in a measurement device reference frame where the effect of misalignment is noticed in the curvature (quadratic form) of the flank position trend, due to "falling-off" of the thread generatrix. This effect is also visible in the increasing separation between flank positions as the Z scan position grows.

To calculate the thread pitch, data must be transformed to the thread reference frame, indicated by $C_2$ in the figure. Data expressed in that frame have a linear trend where flank positions may not be equally spaced but the slope of the linear fit is the thread pitch.

There are two types of pitch that are calculated and obtained by the software:

A first pitch calculated from the slope of the linear fit for all the flanks detected.

A second pitch calculated from the slope of the line formed by two flanks whose separation depends on the thread being analysed (this measurement must be done in relation to the number of threads per inch).

It will be appreciated that the data obtained gives information on the pitch for each longitudinal scan.

The measurement method of the invention is particularly advantageous for measuring threading having a wedge profile, i.e. a profile that has a progressive increase in tooth width and more particularly when the wedge profile is combined to a dove-tail shaped tooth profile in an axial section.

In the case of a wedge thread with a dove-tail tooth profile, the measurement of crests and roots provides for a spiral scan as described above wherein the spiral trajectory follows a line corresponding to the middle position set along the middle distance between the load and stabbing flanks or any other spiral trajectory parallel to said middle position.

In state-of-the-art methods the measuring and controlling of wedge threads provides also that all measurements are taken from a reference point that is conventionally called bolt point. The ball point is defined by passing a bolt, i.e. a measurement element having a small rolling ball of predetermined diameter, along the roots and setting the point where the bolt remains stuck in the root, because the tooth width is variable along the thread. The distance and generatrix at which the bolt point is located with respect to the front of the tube determine the reference point for measuring all parameters of a wedge thread.

According to the measurement method of the invention this operation of setting the bolt point does not need to be performed because the threading parameters are measured from a reference point that is located at the end of the tube.

The determination of this point is based on detecting all load and stabbing flanks in the spatial reference system of the measurement device (X, Y, Z) and then express them in the spatial reference system (X', Y', Z') of the pin using the axes transformation matrix to convert all data retrieved from one spatial reference system to the other spatial reference system, as described above.

Once the flanks positions are expressed in the spatial reference system (X', Y', Z') of the pin, a linear fit is performed on the "Zr-Ur" plane where Zr is the axial position of the flank and Ur is the flank generatrix. This linear fitting is performed for all load and stabbing flanks of the threading separately.

Finally, a subtraction is performed between the two lines adjusted, described in the previous paragraph, and obtaining the "root width" for the whole thread and look for the value (Zr - Ur) of "root valley" where the bolt is stuck in the root for a wedge effect.

The measurement method of the invention for a wedge profile includes also a "Higbee" measurement. The Higbee is conventionally defined as the cut of the first incomplete thread adjacent to the bevel made at the nose of the pin where its intersection with the thread load flank makes a sharp edge. The Higbee corresponds to the removal of the incomplete starting thread (of many types of thread, not only wedge) on tube end, with outer diameter OD $\geq$ 5in. The Higbee removes the starting thread from where thread height is zero, until the thread crest starts, i.e. until where the thread height reaches the acceptance value, and the intersection between the Higbee and the crest of the thread defines a line, parallel to the taper. The arc length of the Higbee is approximately 180º.

Higbee length and height have to meet fabrication tolerances, and depend on the OD and type of connection. Higbee height can be 0, this is to say that the machining tool can reach the root of the thread.

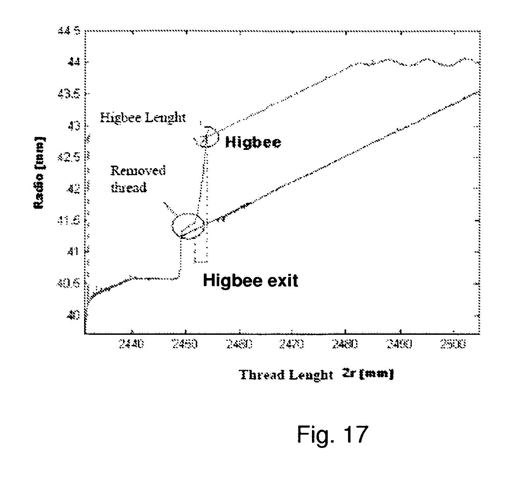

The Higbee point detection is made by adjusting data retrieved from the spiral scan performed on the crest of threading and fitting it to a line and then eliminating from said line all points that are in an area close to the Higbee, when the error between the fitted line and data is greater than some threshold. This point is shown in the graph of figure 17.

The order in which the operations described above are made can vary as well as the number of operations, depending on the necessities and on the parameters to be measured. The completeness of the measurement operation provides also for a calibration of the measurement device before starting operation of the system after set up.

The data points obtained in the longitudinal scan can be further analysed to obtain the value of the step height. Data close to the edges are discarded and a linear fit between consecutive crests is performed, cf. figure 16. The fit is compared to the linear fit of the root and its depth is evaluated by calculating the distance between the line y2 at the center of the root. A similar procedure is performed to calculate the height of the crests, taking y1 as the center of the crest. From the depth and height of the roots and crests, the average and standard deviation are calculated, the incomplete steps are identified and the length of the thread estimated.

Yet another way to calculate the step height, in this case in a global manner, is to subtract a linear fit obtained from the crest and root spiral trajectories. This gives the difference between the inner and outer cones that represents the value of the step height. All variants of the measurement method above described can be applied to a pin having a coating and to a pin before coating. The method of the invention can also be used for measurement of box female threadings, regardless of the presence or absence of a coating, by using an appropriate measurement apparatus of the kind described above and having sensors placed on supports of shape and dimensions appropriate to be inserted inside a pipe.

**Claims**

1. Measurement method of thread parameters, in particular for a threaded object (3), coated by at least one layer of coating material, wherein a measurement device (1) is provided incorporating at least one optical sensor (5', 5") adapted to retrieve the shape of the threaded object, having a nose and defining a first spatial reference system (4) comprising first co-ordinate axes (X', Y', Z'), the measurement device (1) defining a second spatial reference system (2) comprising second co-ordinate axes (X, Y, Z), providing computer means for storing a preset algorithm to calculate a first matrix that describes the quadratic form representing the threaded object in the second spatial reference system, thus providing the relationship between the first and second spatial reference systems, the method comprising, before a coating operation of the threaded object, the steps of:

   a) predefining at least one trajectory of the at least one optical sensor (5', 5") on the threaded object, along which predefined measurement points are selected such that the matrix evaluated on these values has maximum rank,
   b) performing a first scanning operation by the at least one optical sensor (5, 5") along said at least one trajectory and retrieving data of the predefined measurement points,
   c) feeding these data to the preset algorithm and calculating an axes transformation matrix relating the first spatial reference system to the second spatial reference system for defining the relative position of the threaded object with respect to the second spatial reference system,
   d) using the axes transformation matrix to convert all data retrieved from the second spatial reference system to the first spatial reference system, wherein first measurement results are defined,
   e) dismounting the threaded object from the measurement device (1) and, at a subsequent moment in time, performing a coating operation of the threaded object in an appropriate place, wherein at least one layer of coating material is laid on at least part of the object surface, and when the coating operation is performed, mounting the threaded object on the measurement device (1);
   f) performing said method steps from a) to d) on said same at least one trajectory, wherein second measurement results are defined,
   g) comparing first and second measurement results, whereby the thickness of the coating at all predefined measurement points is calculated.

2. Method of claim 1, wherein the first scanning operation by the at least one optical sensor (5', 5") along said at least one trajectory is used to retrieve data for measuring predefined thread parameters.

3. Method of claim 1, wherein one or more second scanning operations are performed on the threaded object, before and after said coating operation, to retrieve data for measuring predefined parameters.

4. Method of claim 2, wherein the at least one trajectory comprises longitudinal scans parallel to the Z axis of the second spatial reference system.

5. Method of claim 3, wherein one or more second scanning operations are performed along helicoidal trajectories.

6. Method of claim 3, wherein during any of the first or second scanning operations the position of at least one thread flank is retrieved.

7. Method of claim 6, wherein a helicoidal thread lead trajectory passing along the middle points of thread crests and/or roots is defined from the position of the at least one thread flank.

8. Method of claim 7, wherein a scanning operation is performed along the helicoidal thread lead trajectory, the retrieved data being fit to a linear dependence.

9. Method of claim 8, wherein a difference between the retrieved data and the linear dependence is obtained and a Fourier analysis is performed on said difference.

10. Method of claim 8, wherein a difference between the retrieved data and the linear dependence is obtained and is evaluated at initial and final portions of the threaded object.

11. Method of claim 8, wherein a difference between the retrieved data and the linear dependence is obtained and is evaluated at any intermediate position between the initial and final portions.

12. Method of claim 8, wherein an alignment of a non machined part of the threaded object with respect to a machined part is evaluated to find relevant process parameters.

13. Method of claim 1, wherein a circular trajectory is defined along the metal-to-metal seal diameter in the first spatial reference system, and then transformed into the second spatial reference system to be executed by the measurement device (1).

14. Method of claim 1, wherein a helicoidal trajectory is defined along the metal-to-metal seal diameter in the first spatial reference system, and then transformed into the second spatial reference system to be executed by the measurement device (1).

15. Method of claim 1, wherein a longitudinal trajectory is defined along the run-in measuring point in the first spatial reference system, and then transformed into the second spatial reference system to be executed by the measurement device (1).

16. Method of claim 1, wherein a longitudinal trajectory is defined along the run-out measuring point in the first spatial reference system, and then transformed into the second spatial reference system to be executed by the measurement device (1).

17. Method of claim 1, wherein at least one longitudinal trajectory intersecting several flanks is determined, the positions of those flanks being retrieved and their longitudinal position being plotted against their angular position, to obtain a linear fit.

18. Method of claim 1, wherein at least one trajectory intersecting thread lead is determined, positions of intersecting points are retrieved, positions of crests and roots are defined and a first linear fit using data from two consecutive crests is made, a second linear fit using data on the root between said two consecutive crests being made and the distance between a first line and a second line respectively defined by the first and second linear fit coefficients being calculated at a predefined point.

19. A measurement device for carrying out the steps a)-d) and f)-g) of the method of claim 1 on a threaded object, comprising:

- at least one computer controlled non-contact laser-based sensor (5', 5"),
- a computer controlled precision mechanical moving system, on which said at least one sensor is mounted, able to guide the at least one sensor (5', 5") during scanning operations according to various scanning patterns,
- a means to synchronize output signals of the at least one sensor (5', 5") with spatial positions of the mechanical system,
- a computer to control the at least one sensor (5', 5") in the scanning operations, to produce computer images of the thread shape of the scanned object, to store the images and to analyse the computer images to obtain quantitative information about thread characteristics like taper, seal diameter and ovality, run-in, run-out, thread diameter, pitch along multiple generatrices of the tube, and step height and coating parameters,
- the computer storing a preset algorithm for calculating an axes transformation matrix, relating a first spatial reference system defined by the threaded object to a second spatial reference system defined by the measurement device, for defining the relative position of the threaded object with respect to the second spatial reference system and for using the axes transformation matrix to convert all data retrieved from the second spatial reference system to the first spatial reference system.

**Patentansprüche**

1. Messverfahren für Gewindeparameter, insbesondere für ein Objekt (3) mit Gewinde, das mit mindestens einer Schicht von Beschichtungsmaterial versehen ist, wobei eine Messvorrichtung (1) vorgesehen ist, die mindestens einen optischen Sensor (5', 5") enthält, welcher dafür ausgelegt ist, die Form des Objektes mit Gewinde wieder zu erfassen, der eine Nase hat und ein erstes räumliches Referenzsystem (4) definiert, welches erste Koordinatenachsen (X', Y', Z') umfasst, wobei die Messvorrichtung (1) ein zweites räumliches Referenzsystem (2) definiert, das zweite Koordinatenachsen (X, Y, Z) umfasst, Computermittel zum Speichern eines vorgegebenen Algorithmus vorsieht, um eine erste Matrix zu berechnen, die die quadratische Form beschreibt, welche das Objekt mit Gewinde im zweiten räumlichen Referenzsystem darstellt, sodass es für die Beziehung zwischen dem ersten und zweiten räumlichen Referenzsystem sorgt, wobei das Verfahren vor einer Beschichtungsoperation des Objektes mit Gewinde folgende Schritte umfasst:

a) Vordefinieren von mindestens einer Bewegungsbahn des mindestens einen optischen Sensors (5', 5") auf dem Objekt mit Gewinde, entlang der vordefinierte Messpunkte so gewählt werden, dass die Matrix, die bei diesen Werten ausgewertet wird, einen maximalen Rang hat,

b) Ausführen einer ersten Scanningoperation durch den mindestens einen optischen Sensor (5', 5") entlang der mindestens einen Bewegungsbahn und Erfassen von Daten der vordefinierten Messpunkte,

c) Einspeisen dieser Daten in den vorgegebenen Algorithmus und Berechnen einer Achsentransformationsmatrix, die das erste räumliche Referenzsystem in Beziehung zum zweiten räumlichen Referenzsystem setzt, um die relative Position des Objektes mit Gewinde bezüglich des zweiten räumlichen Referenzsystems zu definieren,

d) Verwendung der Achsentransformationsmatrix zum Umwandeln aller Daten, die aus dem zweiten räumlichen Referenzsystem abgerufen wurden, in das erste räumliche Referenzsystem, wobei erste Messergebnisse definiert werden,

e) Abnehmen des Objektes mit Gewinde von der Messvorrichtung (1) und in einem anschließenden Moment Ausführen einer Beschichtungsoperation des Objektes mit Gewinde an einem geeigneten Ort, wobei mindestens eine Schicht von Beschichtungsmaterial auf mindestens einen Teil der Objektoberfläche aufgetragen ist, und wenn die Beschichtungsoperation ausgeführt ist, Befestigung des Objektes mit Gewinde auf der Messvorrichtung (1);

f) Ausführen der Verfahrensschritte von a) bis d) auf derselben mindestens einen Bewegungsbahn, wobei zweite Messergebnisse definiert werden,

g) Vergleichen von ersten und zweiten Messergebnissen, wobei die Dicke der Beschichtung an allen vordefinierten Messpunkten berechnet wird.

2. Verfahren nach Anspruch 1, wobei die erste Scanningoperation durch den mindestens einen optischen Sensor (5', 5") entlang der mindestens einen Bewegungsbahn zum Erfassen von Daten zum Messen von vorgegebenen Gewindeparametern verwendet wird.

3. Verfahren nach Anspruch 1, wobei eine oder mehrere zweite Scanningoperationen an dem Objekt mit Gewinde vor und nach der Beschichtungsoperation ausgeführt werden, um Daten zum Messen der vorgegebenen Parameter zu erfassen.

4. Verfahren nach Anspruch 2, wobei die mindestens eine Bewegungsbahn Längsscans parallel zur Z-Achse des zweiten räumlichen Referenzsystems umfasst.

5. Verfahren nach Anspruch 3, wobei eine oder mehrere zweite Scanningoperationen entlang schraubenartiger Bewegungsbahnen ausgeführt werden.

6. Verfahren nach Anspruch 3, wobei während einer der ersten oder zweiten Scanningoperationen die Position von mindestens einer Gewindeflanke erfasst wird.

7. Verfahren nach Anspruch 6, wobei eine schraubenartige Gewindesteigungsbahn, die entlang der Mittelpunkte von Gewindespitzen und/oder Gewindekernen verläuft, aus der Position der mindestens einen Gewindeflanke definiert ist.

8. Verfahren nach Anspruch 7, wobei eine Scanningoperation entlang der schraubenartigen Gewindesteigungsbahn ausgeführt wird, wobei die erfassten Daten an eine lineare Abhängigkeit angepasst werden.

9. Verfahren nach Anspruch 8, wobei eine Differenz zwischen den erfassten Daten und der linearen Abhängigkeit erhalten wird und eine Fourier-Analyse dieser Differenz ausgeführt wird.

10. Verfahren nach Anspruch 8, wobei eine Differenz zwischen den erfassten Daten und der linearen Abhängigkeit erhalten wird und an Anfangs- und Endabschnitten des Gewindeobjektes bewertet wird.

11. Verfahren nach Anspruch 8, wobei eine Differenz zwischen den erfassten Daten und der linearen Abhängigkeit erhalten wird und an einer beliebigen Zwischenposition zwischen dem Anfangs- und Endabschnitt bewertet wird.

**12.** Verfahren nach Anspruch 8, wobei eine Ausrichtung eines nicht bearbeiteten Teils des Gewindeobjektes nach einem bearbeiteten Teil bewertet wird, um relevante Prozessparameter zu finden.

**13.** Verfahren nach Anspruch 1, wobei eine kreisförmige Bewegungsbahn entlang des Metall-Metall-Dichtungsdurchmessers im ersten räumlichen Referenzsystem definiert und dann in das zweite räumliche Referenzsystem transformiert wird, was von der Messvorrichtung (1) ausgeführt werden soll.

**14.** Verfahren nach Anspruch 1, wobei eine schraubenartige Bewegungsbahn entlang des Metall-Metall-Dichtungsdurchmessers im ersten räumlichen Referenzsystem definiert und dann in das zweite räumliche Referenzsystem transformiert wird, was von der Messvorrichtung (1) ausgeführt werden soll.

**15.** Verfahren nach Anspruch 1, wobei eine Längsbahn entlang des Einlaufmesspunktes im ersten räumlichen Referenzsystem definiert und dann in das zweite räumliche Referenzsystem transformiert wird, was von der Messvorrichtung (1) ausgeführt werden soll.

**16.** Verfahren nach Anspruch 1, wobei eine Längsbahn entlang des Auslaufmesspunktes im ersten räumlichen Referenzsystem definiert und dann in das zweite räumliche Referenzsystem transformiert wird, was von der Messvorrichtung (1) ausgeführt werden soll.

**17.** Verfahren nach Anspruch 1, wobei mindestens eine Längsbahn, die mehrere Flanken schneidet, bestimmt wird, wobei die Positionen dieser Flanken erfasst und ihre Längsposition über ihrer Winkelposition aufgetragen werden, um einen linearen Ausgleich zu erhalten.

**18.** Verfahren nach Anspruch 1, wobei mindestens eine Bewegungsbahn, die die Gewindesteigung schneidet, bestimmt wird, Positionen von Schnittpunkten erfasst werden, Positionen von Gewindespitzen und -kernen definiert werden und eine erste lineare Anpassung unter Verwendung von Daten von zwei aufeinanderfolgenden Gewindespitzen erzeugt wird, eine zweite lineare Anpassung unter Verwendung von Daten am Gewindekern zwischen zwei aufeinanderfolgenden Gewindespitzen erzeugt wird und die Distanz zwischen einer ersten Linie und einer zweiten Linie, die durch die ersten bzw. zweiten linearen Anpassungskoeffizienten definiert sind, an einem vorgegebenen Punkt berechnet wird.

**19.** Messvorrichtung zum Ausführen der Schritte a)-d) und f)-g) des Verfahrens nach Anspruch 1 an einem Objekt mit Gewinde, die Folgendes umfasst:

• mindestens einen computergesteuerten kontaktlosen Sensor auf Laserbasis (5', 5"),
• ein computergesteuertes mechanisches Präzisionsbewegungssystem, auf dem der mindestens eine Sensor befestigt ist, das den mindestens einen Sensor (5', 5") während der Scanningoperationen gemäß verschiedener Scanningparameter führen kann,
• Mittel zum Synchronisieren der Ausgangssignale des mindestens einen Sensors (5', 5") mit räumlichen Positionen des mechanischen Systems,
• einen Computer zum Steuern des mindestens einen Sensors (5', 5") bei den Scanningoperationen, um Computerbilder der Gewindeform des gescannten Objektes zu erzeugen, die Bilder zu speichern und die Computerbilder zu analysieren, um quantitative Informationen über Gewindemerkmale zu erhalten, wie Gewindekegel, Abdichtungsdurchmesser und Ovalität, Einlauf, Auslauf, Gewindedurchmesser, Steigung entlang mehrerer Erzeugenden des Rohres und Stufenhöhe und Beschichtungsparameter,
• wobei der Computer einen vorgegebenen Algorithmus zum Berechnen einer Achsentransformationsmatrix speichert, ein erstes räumliches Referenzsystem, das durch das Objekt mit Gewinde definiert ist, in Beziehung zu einem zweiten räumlichen Referenzsystem setzt, welches durch die Messvorrichtung definiert ist, um die relative Position des Objektes mit Gewinde in Bezug auf das zweite räumliche Referenzsystem zu definieren, und um mit der Achsentransformationsmatrix alle Daten, die aus dem zweiten räumlichen Referenzsystem abgerufen wurden, in das erste räumliche Referenzsystem zu konvertieren.

**Revendications**

**1.** Procédé de mesure de paramètres de filetage, en particulier pour un objet fileté (3), revêtu d'au moins une couche de matériau de revêtement, dans lequel il est fourni un dispositif de mesure (1) incorporant au moins un capteur optique (5', 5") apte à extraire la forme de l'objet fileté, ayant un nez et définissant un premier système de référence spatiale (4) comprenant des premiers axes de coordonnées (X', Y', Z'), le dispositif de mesure (1) définissant un deuxième système de référence spatiale (2) comprenant des deuxièmes axes de coordonnées (X, Y, Z), fournissant un moyen informatique pour stocker un algorithme préréglé pour calculer une première matrice qui décrit la forme quadratique représentant l'objet fileté dans le deuxième système de référence spatiale, en fournissant ainsi la relation entre le premier système de référence spatiale et le deuxième système de référence spatiale, le procédé comprenant, avant une opération de revêtement de l'objet fileté, les étapes suivantes :

a) la prédéfinition d'au moins une trajectoire de l'au moins un capteur optique (5', 5 ") sur l'objet fileté, le long de laquelle des points de mesure prédéfinis sont sélectionnés de sorte que la matrice évaluée sur ces valeurs ait un rang maximal,

b) l'exécution d'une première opération de balayage par l'au moins un capteur optique (5, 5") le long de ladite au moins une trajectoire et l'extraction de données des points de mesure prédéfinis,

c) l'alimentation de ces données dans l'algorithme préréglé et le calcul d'une matrice de transformation d'axes mettant le premier système de référence spatiale en relation avec le deuxième système de référence spatiale pour définir la position relative de l'objet fileté par rapport au deuxième système de référence spatiale,

d) l'utilisation de la matrice de transformation d'axes pour convertir toutes les données extraites du deuxième système de référence spatiale dans le premier système de référence spatiale, dans lequel des premiers résultats de mesure sont définis,

e) le démontage de l'objet fileté du dispositif de mesure (1) et, à un moment ultérieur, l'exécution d'une opération de revêtement de l'objet fileté à un emplacement approprié, dans lequel au moins une couche de matériau de revêtement est posée sur au moins une partie de la surface d'objet, et lorsque l'opération de revêtement est exécutée, le montage de l'objet fileté sur le dispositif de mesure (1) ;

f) l'exécution desdites étapes de procédé de a) à d) sur ladite même au moins une trajectoire, dans lequel des deuxièmes résultats de mesure sont définis, g) la comparaison des premiers et deuxièmes résultats de mesure, au moyen de laquelle l'épaisseur du revêtement à tous les points de mesure prédéfinis est calculée.

2. Procédé selon la revendication 1, dans lequel la première opération de balayage par l'au moins un capteur optique (5', 5 ") le long de ladite au moins une trajectoire est utilisée pour extraire des données pour mesurer des paramètres de filetage prédéfinis.

3. Procédé selon la revendication 1, dans lequel une ou plusieurs deuxièmes opérations de balayage sont effectuées sur l'objet fileté, avant et après ladite opération de revêtement, pour extraire des données pour mesurer des paramètres prédéfinis.

4. Procédé selon la revendication 2, dans lequel l'au moins une trajectoire comprend des balayages longitudinaux parallèles à l'axe Z du deuxième système de référence spatiale.

5. Procédé selon la revendication 3, dans lequel une ou plusieurs deuxièmes opérations de balayage sont effectuées le long de trajectoires hélicoïdales.

6. Procédé selon la revendication 3, dans lequel, au cours de la première ou de la deuxième opération de balayage, la position de l'au moins un flanc de filetage est extraite.

7. Procédé selon la revendication 6, dans lequel une trajectoire hélicoïdale de pas de filetage passant par les points de milieu de crêtes et/ou de racines de filetage est définie à partir de la position de l'au moins un flanc de filetage.

8. Procédé selon la revendication 7, dans lequel une opération de balayage est effectuée le long de la trajectoire hélicoïdale de pas de filetage, les données extraites étant mises en correspondance avec une dépendance linéaire.

9. Procédé selon la revendication 8, dans lequel une différence entre les données extraites et la dépendance linéaire est obtenue et une analyse de Fourier est effectuée sur ladite différence.

10. Procédé selon la revendication 8, dans lequel une différence entre les données extraites et la dépendance linéaire est obtenue et est évaluée à une portion initiale et à une portion finale de l'objet fileté.

11. Procédé selon la revendication 8, dans lequel une différence entre les données extraites et la dépendance linéaire est obtenue et est évaluée à une position intermédiaire entre la portion initiale et la portion finale.

**12.** Procédé selon la revendication 8, dans lequel un alignement d'une partie non usinée de l'objet fileté par rapport à une partie usinée est évalué pour trouver des paramètres de traitement pertinents.

**13.** Procédé selon la revendication 1, dans lequel une trajectoire circulaire est définie le long du diamètre de joint de métal sur métal dans le premier système de référence spatiale, puis transformée dans le deuxième système de référence spatiale pour être exécutée par le dispositif de mesure (1).

**14.** Procédé selon la revendication 1, dans lequel une trajectoire hélicoïdale est définie le long du diamètre de joint de métal sur métal dans le premier système de référence spatiale, puis transformée dans le deuxième système de référence spatiale pour être exécutée par le dispositif de mesure (1).

**15.** Procédé selon la revendication 1, dans lequel une trajectoire longitudinale est définie le long du point de mesure de sillon d'entrée dans le premier système de référence spatiale, puis transformée dans le deuxième système de référence spatiale pour être exécutée par le dispositif de mesure (1).

**16.** Procédé selon la revendication 1, dans lequel une trajectoire longitudinale est définie le long du point de mesure de sillon de sortie dans le premier système de référence spatiale, puis transformée dans le deuxième système de référence spatiale pour être exécutée par le dispositif de mesure (1).

**17.** Procédé selon la revendication 1, dans lequel il est déterminé au moins une trajectoire longitudinale en intersection avec plusieurs flancs dont les positions sont extraites et dont la position longitudinale est tracée par rapport à la position angulaire, pour obtenir une adéquation linéaire.

**18.** Procédé selon la revendication 1, dans lequel il est déterminé au moins une trajectoire en intersection avec le pas de filetage, les positions des points d'intersection sont extraites, les positions des crêtes et des racines sont définies et une première adéquation linéaire utilisant des données de deux crêtes consécutives est effectuée, une deuxième adéquation linéaire utilisant des données sur la racine entre lesdites deux crêtes consécutives est effectuée et la distance entre une première ligne et une deuxième ligne respectivement définies par les premier et deuxième coefficients d'adéquation linéaire est calculée à un point prédéfini.

**19.** Dispositif de mesure pour effectuer les étapes a)-d) et f)-g) du procédé de la revendication 1 sur un objet fileté, comprenant :

- au moins un capteur à base de laser sans contact commandé par ordinateur (5', 5 "),
- un système de déplacement mécanique de précision commandé par ordinateur, sur lequel ledit au moins un capteur est monté, capable de guider l'au moins un capteur (5', 5") au cours d'opérations de balayage selon divers motifs de balayage,
- un moyen pour synchroniser des signaux de sortie de l'au moins un capteur (5', 5 ") avec des positions spatiales du système mécanique,
- un ordinateur pour commander l'au moins un capteur ( 5' , 5 " ) au cours des opérations de balayage, pour produire des images informatiques de la forme de filetage de l'objet balayé, pour stocker les images et pour analyser les images informatiques afin d'obtenir des informations quantitatives sur des caractéristiques de filetage comme le taraudage, le diamètre et l'ovalité de joint, le sillon d'entrée, le sillon de sortie, le diamètre de filetage, le pas le long de plusieurs génératrices du tube, et des paramètres de revêtement et de hauteur de marche,
- l'ordinateur stockant un algorithme préréglé pour calculer une matrice de transformation d'axes, mettant un premier système de référence spatiale défini par l'objet fileté en relation avec un deuxième système de référence spatiale défini par le dispositif de mesure, pour définir la position relative de l'objet fileté par rapport au deuxième système de référence spatiale et pour utiliser la matrice de transformation d'axes afin de convertir toutes les données extraites du deuxième système de référence spatiale dans le premier système de référence spatiale.

Fig. 1

Fig. 1a

```
┌─────────────────┐            ┌─────────────────┐
│   Move pipe     │            │  Reset position │
└────────┬────────┘            └────────┬────────┘
         │                              │
         ▼                              ▼
┌─────────────────┐            ┌─────────────────┐
│ Verify pipe in  │──────────▶ │  Nose detection │
│    position     │            └────────┬────────┘
└─────────────────┘                     │
                                        ▼
                             ┌─────────────────┐      ┌──────────────────────┐
                             │ Set scan limits │◀────▶│ Read value of Lc in   │
                             └────────┬────────┘      │        file          │
                                      │               └──────────────────────┘
                                      ▼
┌─────────────────┐          ┌─────────────────┐
│ Condition in    │◀───────▶ │ Thread          │
│    signal       │          │ longitudinal    │
└─────────────────┘          │    scan         │
                             └────────┬────────┘
                                      │
                                      ▼
                             ┌─────────────────┐      ┌──────────────────────┐
                             │ Find thread     │◀────▶│ Read value of pitch  │
                             │ angular and     │      │ and step height in   │
                             │ lateral         │      │        file          │
                             │ deviations      │      └──────────────────────┘
                             └────────┬────────┘
                                      │
                                      ▼
                             ┌─────────────────┐
                             │ Set Valley and  │
                             │ crest trajectory│
                             └────────┬────────┘
                                      │
                                      ▼
┌─────────────────┐          ┌─────────────────┐
│ Condition in    │◀───────▶ │ Thread spiral   │
│    signal       │          │ scan througt    │
└─────────────────┘          │    valley       │
                             └────────┬────────┘
                                      │
                                      ▼
┌─────────────────┐          ┌─────────────────┐
│ Condition in    │◀───────▶ │ Thread spiral   │
│    signal       │          │ scan througt    │
└─────────────────┘          │    crest        │
                             └────────┬────────┘
                                      │
                                      ▼
                             ┌─────────────────┐
                             │ Set seal        │
                             │ diameter        │
                             │ trajectory      │
                             └────────┬────────┘
                                      │
                                      ▼
┌─────────────────┐          ┌─────────────────┐
│ Condition in    │◀───────▶ │ Set seal        │
│    signal       │          │ diameter        │
└─────────────────┘          └────────┬────────┘
                                      │
                                      ▼
┌─────────────────┐          ┌─────────────────┐
│   Move pipe     │◀──────── │ Go to rest      │
└────────┬────────┘          │   position      │
         │                   └────────┬────────┘
         ▼                            │
┌─────────────────┐                   ▼
│ Verify no pipe  │          ┌─────────────────┐      ┌──────────────────────┐
│    position     │          │ Data Analysis   │◀────▶│ Read nominal thread  │
└─────────────────┘          └─────────────────┘      │   values from file   │
                                                      └──────────────────────┘
```

| Taber | Run-out | Pipe ovality | Step heigth |
| Run-in | Pipe diameter | Pitch | Seal diameter |
| | | | Seal Ovality |

## Fig. 2

20

ts [mm]

65

60

55

50

305   310   315   320   325   330   335   340   345

Zs axis [mm]

Fig. 3

Nose
margin

Measurement
window

Rest position

W$_{m1}$

Nose position

W$_{m2}$

Z$_0$ security

Fig. 3a

Fig. 4

Fig. 5

Fig. 6

(a)

(b)

(c)

(d)

(e)

(f)

Fig. 7

(a)

(b)

Fig. 8

(a)

(b)

Fig. 9

Fig. 10

Fig. 11

Fig. 12

Fig. 13

Fig. 14

Fig. 15

Fig. 16

Fig. 17

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1554518 A
- EP 1954953 A
- EP 2102542 A
- US 5521707 A